# EUROPEAN PATENT SPECIFICATION

(11) **EP 1 713 769 B1**
(45) Date of publication and mention of the grant of the patent: **24.06.2009**
(21) Application number: 05715224.1
(22) Date of filing: 28.01.2005
(51) Int. Cl.: C07C 311/37, A61K 31/18, A61P 35/00

(54) **AMORPHOUS TAMSULOSIN HYDROCHLORIDE**
AMORPHES TAMSULOSIN-HYDROCHLORID
CHLORHYDRATE DE TAMSULOSINE AMORPHE

(30) Priority: 29.01.2004 SI 200400032
(43) Date of publication of application: 25.10.2006
(73) Proprietor: LEK Pharmaceuticals d.d., 1526 Ljubljana (SI)
(72) Inventor: HAM, Zoran, 1420 Trbovlje (SI)
(74) Representative: Kröger, Bernd
(86) International application number: PCT/EP2005/000875
(87) International publication number: WO 2005/075416

(56) References cited:
- EP-A- 0 257 787
- WO-A-03/037850
- WO-A-03/039530

## Description

### FIELD OF THE INVENTION

The present invention relates to the amorphous form of (R)-5-(2-(2-(2-ethoxyphenoxy)ethylamino)propyl)-2-methoxybenzenesulphonamide hydrochloride, known under the generic name tamsulosin hydrochloride, and to preparation of the amorphous form of high purity.

### BACKGROUND OF THE INVENTION

(R)-5-(2-(2-(2-ethoxyphenoxy)ethylamino)propyl)-2-methoxybenzenesulphon amide hydrochloride (formula 1), designated with the generic pharmaceutical name tamsulosin hydrochloride, is used in the form of pure R-enantiomer for the treatment of benign prostatic hyperplasia.

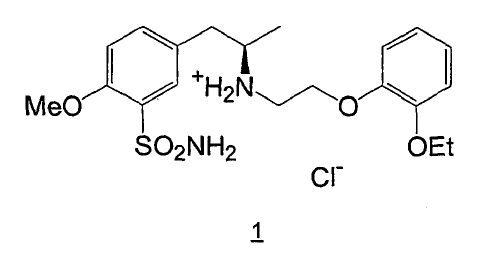

For incorporation into a pharmaceutical formulation, pharmaceutical active substances must show defined desired physico-chemical properties, such as solubility in water and certain solvents, suitable particle size, stability and non-hygroscopicity. All of these properties can be regulated through use of different crystalline forms. Thereby optimal bioavailability can be achieved.

Different crystalline forms can be identified by physico-chemical methods that measure parameters dependent on the molecular environment. The most useful methods are X-ray powder diffraction, infrared (IR) spectroscopy and differential scanning calorimetry (DSC).

In practice, the preferred method for distinguishing an amorphous from a crystalline form is X-ray powder diffraction. An amorphous form is characterised on the basis of the absence of diffractions at all angles in the X-ray powder diffractogram thereof.

In WO 03/039530, tablets of Tamsulosin.HCl are disclosed. No mention of the polymorph could be found therein however the use of crystalline Tamsulosin, as known at that time, can be assumed.

Unlike the tamsulosin base, tamsulosin in the form of the hydrochloride salt has not been known to exist in different crystal forms. Thus the amorphous form of tamsulosin hydrochloride has not been described to date.

### SUMMARY OF THE INVENTION

In the first embodiment, the invention concerns tamsulosin hydrochloride in the amorphous form.

In another embodiment, the invention concerns a process for preparation of amorphous tamsulosin hydrochloride by lyophilization of a solution of tamsulosin hydrochloride.

In another embodiment, the invention concerns a process for preparation of amorphous tamsulosin hydrochloride by spray-drying of a solution of tamsulosin hydrochloride.

In another embodiment, the invention concerns a pharmaceutical formulation comprising amorphous tamsulosin hydrochloride and one or more pharmaceutically acceptable excipients.

In another embodiment, the invention concerns the use of amorphous tamsulosin hydrochloride in the preparation of a medicament for the prevention and treatment of benign prostatic hyperplasia.

### DESCRIPTION OF THE FIGURES

- *Figure 1:*: Dashed line: DSC curve of crystalline tamsulosin hydrochloride. Solid line: DSC curve of amorphous tamsulosin hydrochloride. A differential scanning calorimeter Mettler Toledo DSC822^{e} was used. Measurements were performed in open pots with a heating rate 3 °C/min with blowing of nitrogen.
- *Figure 2:*: IR spectrum of crystalline tamsulosin hydrochloride. An infrared spectrometer »Bio-Rad FTS-60, Digilab-Division« was used.
- *Figure 3:*: IR spectrum of amorphous tamsulosin hydrochloride obtained as for Figure 2.
- *Figure 4:*: Above: X-ray powder diffractogram of amorphous tamsulosin hydrochloride. Below: X-ray powder diffractogram of crystalline tamsulosin hydrochloride. A Philips PW 1710 with the reflection technique under the conditions: CuKa radiation, range of 2° to 37° 20, step 0.04° 2θ, and integration time 1 s was used.

### DETAILED DESCRIPTION OF THE INVENTION

In the present invention, the formation of a completely amorphous form of tamsulosin hydrochloride is disclosed, i.e. the amorphous tamsulosin hydrochloride is preferably substantially pure, for instance at least 75 % pure, or at least 90 % pure, or at least 95 % pure. Preferably pharmaceutical formulations according to the invention comprising the amorphous form of tamsulosin hydrochloride are substantially free of other physical forms of tamsulosin hydrochloride. Optionally the amorphous form may co-exist with other physical forms in pharmaceutical formulations.

Tamsulosin and/or tamsulosin hydrochloride used as a precursor in the preparation of amorphous tamsulosin hydrochloride may be synthesised by any process as described elsewhere, such as in EP 34432.

In view of the aforementioned description of the significance of physico-chemical properties of the active substance for preparation of a final pharmaceutical formulation to reach a suitable bioavailability for a desired dosing, we have experimented with the preparation of tamsulosin with different physico-chemical properties. It has been found that by using different solvents, particles with minimal size variability can be obtained, which may later be adjusted to a desired size for manufacturing a pharmaceutical formulation by milling alone, whereas no variation in the crystal form can be observed.

Surprisingly, we have found that by lyophilization of an aqueous solution of tamsulosin hydrochloride, a physico-chemical form is obtained which is distinct from known forms, namely a fine amorphous powder. Using this short, simple and robust method of preparation, tamsulosin hydrochloride having suitable physico-chemical properties for incorporation into a pharmaceutical formulation can be prepared without undergoing a long and rigorously controlled crystallisation process. The amorphous form usually shows improved bioavailability over the crystalline form. The problem of undesirable solvent residues has been solved by using water as a solvent in the final phase.

The amorphous product of the lyophilization technique disclosed herewith is sufficiently pure to use directly in formulating pharmaceutical-grade compositions.

According to one method of preparing amorphous tamsulosin hydrochloride, crystalline tamsulosin hydrochloride is dissolved in a solvent, in water, to obtain a concentration of about 0.5 g/l to about 5 g/I, preferably about 1 g/l to about 5 g/l, most preferably about 5 g/l at room temperature. The obtained solution can be frozen using liquid nitrogen. The frozen solution can be lyophilised from about 12 to about 56 hours, preferably about 24 - 48 hours, most preferably about 36 hours.

Alternatively amorphous tamsulosin hydrochloride can be prepared by spray-drying. Crystalline tamsulosin hydrochloride is dissolved in a solvent, wherein tamsulosin is soluble, for instance in water or in a mixture of water and an organic solvent, preferably methanol, to obtain a saturated solution at room temperature. The obtained solution can be spray-dried. The product is amorphous tamsulosin hydrochloride.

The prepared amorphous form of tamsulosin hydrochloride can be characterised by the following physico-chemical methods:
a. Differential scanning calorimetry,
b. Melting point determination,
c. IR spectroscopy and
d. X-ray powder diffraction.

### a. Differential scanning calorimetry

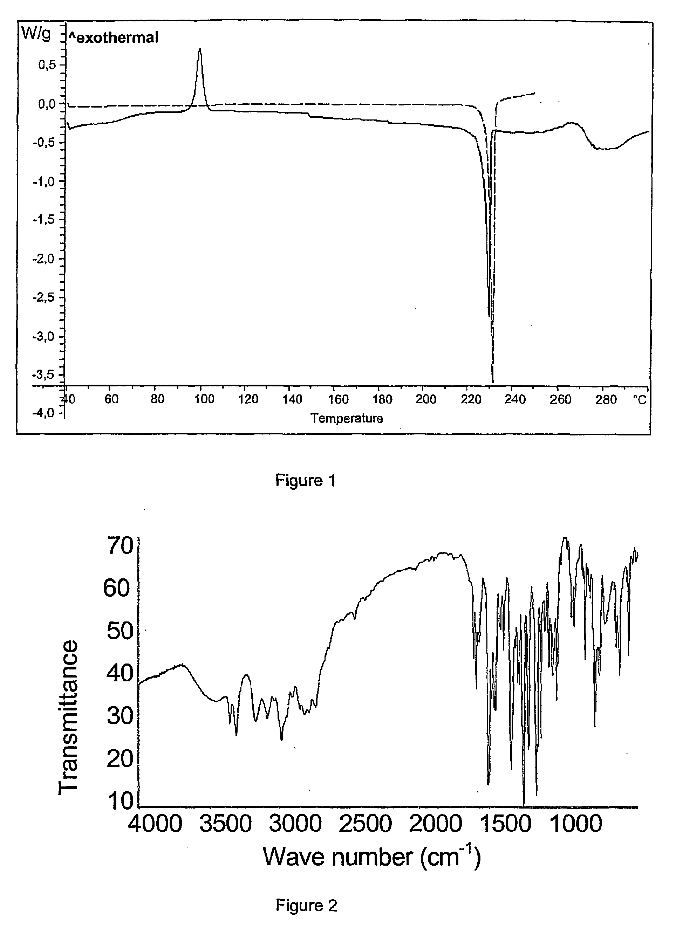

A differential scanning calorimeter Mettler Toledo DSC822^{e} or other suitable equipment can be used. For instance, when measurements are performed using this equipment in open pots with a heating rate 3 °C/min with blowing of nitrogen, crystalline tamsulosin hydrochloride exhibits one endothermic peak at 230 °C, which is the melting point (Figure 1, dashed line). The amorphous form shows an exothermic peak on the thermogram at 100 °C, which represents crystallization of the amorphous form into the crystalline form with the additional endothermic peak at 230 °C which represents the melting point (Figure 1, solid line).

### b. Melting point

The melting point can be determined, for instance, by the method of visual inspection during heating on a microscope with a heated table Leica Thermogalen.

Before melting, the amorphous form is converted into the crystalline form (as is also indicated by DSC). Thus the melting point of the amorphous tamsulosin hydrochloride does not essentially differ from the melting point of crystalline tamsulosin hydrochloride - both are melted in the interval between 226 and 228 °C.

### c. IR spectroscopy

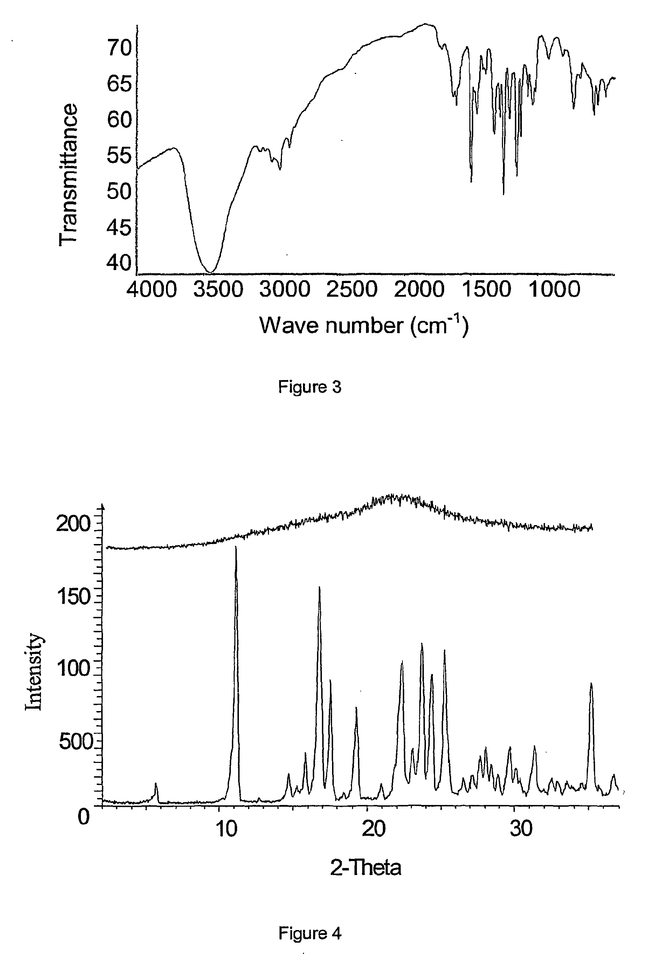

The respective IR spectra are shown in Figures 2 and 3. The most distinct absorption bands between 3500 and 700 cm⁻¹ when measured using, for instance a Bio Rad FTS 60, Digilab Division infrared spectrometer are shown in the following table 1:

**Table 1: Bands in the IR spectra of the crystalline and the amorphous tamsulosin hydrochloride, respectively, measured using, for instance a Bio Rad FTS 60, Digilab Division infrared spectrometer**

| Crystalline tamsulosin hydrochloride (cm⁻¹) | Amorphous tamsulosin hydrochloride (cm⁻¹) |
|---|---|
| - | 3449 |
| 3355 | - |
| 3308 | - |
| 3167 | - |
| 3084 | - |
| 2983 | 2926 |
| 1610 | 1609 |
| 1506 | 1504 |
| 1499 | 1497 |
| 1339 | 1328 |
| 1251 | 1255 |
| 1215 | 1213 |
| 1160 | 1159 |
| 1128 | 1128 |
| 1018 | - |
| 819 | - |
| 748 | 752 |
| 717 | - |

IR spectra of the amorphous and crystalline forms of tamsulosin hydrochloride mainly differ in the intensities of the absorption bands. In the amorphous form, absorption bands are wider and rounded and the absence of many bands is observed compared to the crystalline form. That is particularly characteristic in the regions of 1000 - 1100 cm⁻¹ and 1450 - 1500 cm⁻¹. An essential difference is at 3450 cm⁻¹ where in the amorphous form, there is only one distinctly broad adsorption band whereas in the crystalline form, there are several less distinct bands.

### d. X-ray powder diffraction analysis

An X-ray powder diffractogram of the amorphous form of tamsulosin hydrochloride indicates the absence of discrete diffractions which are characteristic of crystalline forms and continuous diffraction over the entire scanned region which is the confirmation of an amorphous nature of the material. Both diffractograms are shown in Figure 4, respectively, which were produced using a Philips PW 1710 with the reflection technique under the following conditions: CuKα radiation, range of 2° to 37° 2θ, step 0.04° 2θ, and integration time 1 s.

The most distinct bands of the crystalline form are at the following 2θ: 11.0, 14.5, 15.2, 15.7, 16.7, 17.5, 19.2, 20.8, 22.3, 23.0, 23.5, 24.3 and 25.2.

The present invention also provides the use of amorphous physical form of tamsulosin hydrochloride in the manufacture of a pharmaceutical formulation, optionally in a mixture with other forms of tamsulosin hydrochloride. The pharmaceutical formulations prepared from amorphous tamsulosin hydrochloride may be used in a manner similar to that of a pharmaceutical formulation prepared from any other existing forms of tamsulosin hydrochloride. Preferably a pharmaceutical formulation can be prepared by combining amorphous tamsulosin hydrochloride with one or more pharmaceutically acceptable excipients. Preferably an excipient may be a binder, a filler, a diluent, a spheronizing agent, a lubricant, a release control agent or other.

The pharmaceutical formulation comprises a therapeutically effective amount of tamsulosin hydrochloride. As used herein, the term "effective amount" refers to the amount or dose of the compound which provides the desired effect in the patient under treatment, upon single or multiple dose administration.

The pharmaceutical formulation can be administered preferably orally but other ways of administration may be also possible.

The pharmaceutical formulation may be in the form of a pellet, capsule, tablet, powder, granulate or any other suitable form, preferably in the form of pellets in capsules.

The active ingredient and excipients may be formulated into pharmaceutical formulations according to methods known in the art.

Tamsulosin hydrochloride produced by the process of the present invention and formulated accordingly can be then used for the prevention and/or treatment of benign prostatic hyperplasia.

According to the present invention there is provided a method of treating benign prostatic hyperplasia which comprises administering a therapeutically effective amount of amorphous tamsulosin hydrochloride in conjunction with a pharmaceutically acceptable diluent or carrier.

### EXAMPLES

The present invention is illustrated but in no way limited by the following examples:

### Example 1

### 5-(2-(2-(2-ethoxyphenoxy)-ethylamino)-propyl)-2-methoxybenzenesulphon amide hydrochloride (tamsulosin hydrochloride)

10 g of (R)-2-[N-(trifluoroacetyl)amino]-1-(4'-methoxy-3'-sulphamoyl)phenyl propane, 19 g of 2-(-o-ethoxyphenoxy)ethyl bromide and 170 ml of MeOH are refluxed for 43 hours. MeOH is evaporated *in vacuo* on a rotavapor at 60 °C. 170 ml of water and 130 ml of ethyl acetate are added to the residue, and while cooling and stirring, 16 g of 50 % NaOH is added. If both phases are not clear, NaOH is further added until clarification. After separation of both phases, the water phase is further extracted with 2 x 100 ml of ethyl acetate. Combined extracts are washed with 2 x 130 ml of water and evaporated *in vacuo* on a rotavapor at 60 °C. The obtained product is dissolved in 100 ml of EtOH and while cooling and stirring, 7 ml of ethanolic HCl (approx. 300 mg HCl/ml) is added. During cooling (0 °C) it is stirred for 4 hours and the formed crude tamsulosin hydrochloride is centrifuged and washed with 20 ml of cold EtOH and dried *in vacuo* at 40 °C. 7.0 g of a product is obtained which may be additionally purified by crystallization from methanol.

### Example 2

### Preparation of amorphous tamsulosin hydrochloride by lyophilization

1.0 g of crystalline tamsulosin hydrochloride is dissolved in 200 ml of water. To speed up the dissolution, the mixture may be gently heated. The resulting clear solution is filtered and frozen. The frozen mixture is lyophilized for 36 hours. Fine white powder is obtained.

### Example 3

### Preparation of amorphous tamsulosin hydrochloride by spray-drying

1.5 g of crystalline tamsulosin hydrochloride is dissolved in 50 ml of water and 25 ml of methanol. The resulting solution is filtered and spray-dried by inlet temperature of air of 150 °C. Fine white powder is obtained.

## Claims

1. Tamsulosin hydrochloride, ((R)-5-(2-(2-(2-ethoxyphenoxy)ethy)amino) propyl)-2-methoxybenzenesulphonamide) hydrochloride, in the amorphous form.

2. Tamsulosin hydrochloride in the amorphous form according to claim 1 **characterised in that** the DSC thermogram thereof exhibits an exothermic peak at 100 °C.

3. Tamsulosin hydrochloride in the amorphous form according to claim 1 **characterised in that** the IR spectrum thereof exhibits a band at 3449 cm⁻¹.

4. Tamsulosin hydrochloride in the amorphous form according to claim 3 **characterised in that** the IR spectrum thereof additionally exhibits the bands at one or more of the 752, 1128, 1159, 1213, 1255, 1328, 1497, 1504, 1609 and 2926 cm⁻¹.

5. A process for the preparation of the amorphous form of tamsulosin hydrochloride **characterised in that** it comprises lyophilization of a solution of tamsulosin hydrochloride.

6. A process for the preparation of the amorphous form of tamsulosin hydrochloride **characterised in that** it comprises spray-drying of a solution of tamsulosin hydrochloride.

7. A pharmaceutical formulation comprising tamsulosin hydrochloride and one or more pharmaceutically acceptable excipients **characterised in that** it comprises tamsulosin hydrochloride in the amorphous form.

8. The formulation according to claim 7 **characterised in that** said tamsulosin hydrochloride is prepared by the processes according to claims 5 or 6.

9. A method of preparing a pharmaceutical formulation of tamsulosin hydrochloride, in the amorphous form comprising combining an amount of tamsulosin hydrochloride in the amorphous form with pharmaceutically acceptable excipients.

10. Use of tamsulosin hydrochloride in the amorphous form for the preparation of a medicament for the treatment of benign prostatic hyperplasia.

## Patentansprüche

1. Tamsulosinhydrochlorid ((R)-5-(2-(2-(2-Ethoxyphenoxy)-ethylamino)-propyl)-2-methoxybenzol-sulfonamid)-hydrochlorid in der amorphen Form.

2. Tamsulosinhydrochlorid in der amorphen Form nach Anspruch 1, **dadurch gekennzeichnet, dass** das DSC Thermogramm hiervon bei 100 °C einen exothermen Peak zeigt.

3. Tamsulosinhydrochlorid in der amorphen Form nach Anspruch 1, **dadurch gekennzeichnet, dass** das IR Spektrum hiervon bei 3449 cm⁻¹ eine Bande zeigt.

4. Tamsulosinhydrochlorid in der amorphen Form nach Anspruch 3, **dadurch gekennzeichnet, dass** das IR Spektrum hiervon zusätzlich bei 752, 1128, 1159, 1213, 1255, 1328, 1497, 1504, 1609 und 2926 cm⁻¹ ein oder mehr Banden zeigt.

5. Verfahren zur Herstellung der amorphen Form von Tamsulosinhydrochlorid, **dadurch gekennzeichnet, dass** es eine Lyophilisation einer Lösung von Tamsulosinhydrochlorid umfasst.

6. Verfahren zur Herstellung der amorphen Form von Tamsulosinhydrochlorid, **dadurch gekennzeichnet, dass** es eine Sprühtrocknung einer Lösung von Tamsulosinhydrochlorid umfasst.

7. Pharmazeutische Formulierung, die Tamsulosinhydrochlorid und ein oder mehr pharmazeutisch akzeptable Hilfsstoffe umfasst, **dadurch gekennzeichnet, dass** diese Formulierung Tamsulosinhydrochlorid in der amorphen Form umfasst.

8. Formulierung nach Anspruch 7, **dadurch gekennzeichnet, dass** das Tamsulosinhydrochlorid durch die Verfahren nach den Ansprüchen 5 oder 6 hergestellt wird.

9. Verfahren zur Herstellung einer pharmazeutischen Formulierung von Tamsulosinhydrochlorid in der amorphen Form, umfassend eine Kombination einer Menge von Tamsulosinhydrochlorid in der amorphen Form mit pharmazeutisch akzeptablen Hilfsstoffen.

10. Verwendung von Tamsulosinhydrochlorid in der amorphen Form zur Herstellung eines Arzneimittels für die Behandlung benigner Prostatahyperplasie.

## Revendications

1. Chlorhydrate de tamsulosine, chlorhydrate de (R)-5-(2-(2-(2-éthoxyphénoxy)éthylamino)propyl)-2-méthoxybenzènesulfonamide, sous forme amorphe.

2. Chlorhydrate de tamsulosine sous forme amorphe selon la revendication 1, **caractérisé en ce que** son thermogramme d'analyse calorimétrique différentielle révèle un pic exothermique à 100°C.

3. Chlorhydrate de tamsulosine sous forme amorphe selon la revendication 1, **caractérisé en ce que** son spectre infra-rouge révèle une bande à 3449 cm⁻¹.

4. Chlorhydrate de tamsulosine sous forme amorphe selon la revendication 3, **caractérisé en ce que** son spectre infra-rouge révèle en plus une ou plusieurs des bandes suivantes, à 752, 1128, 1159, 1213, 1255, 1328, 1497, 1504, 1609 et 2926 cm⁻¹.

5. Procédé de préparation de la forme amorphe du chlorhydrate de tamsulosine, **caractérisé en ce qu'**il comprend la lyophilisation d'une solution de chlorhydrate de tamsulosine.

6. Procédé de préparation de la forme amorphe du chlorhydrate de tamsulosine, **caractérisé en ce qu'**il comprend le séchage par atomisation d'une solution de chlorhydrate de tamsulosine.

7. Formulation pharmaceutique comprenant du chlorhydrate de tamsulosine et un ou plusieurs excipients pharmaceutiquement acceptables, **caractérisée en ce qu'**elle comprend le chlorhydrate de tamsulosine sous forme amorphe.

8. Formulation selon la revendication 7, **caractérisée en ce que** ledit chlorhydrate de tamsulosine est préparé au moyen du procédé selon la revendication 5 ou 6.

9. Procédé de préparation d'une formulation pharmaceutique de chlorhydrate de tamsulosine sous forme amorphe, comprenant la combinaison d'une certaine quantité de chlorhydrate de tamsulosine sous forme amorphe avec des excipients pharmaceutiquement acceptables.

10. Utilisation de chlorhydrate de tamsulosine sous forme amorphe pour la préparation d'un médicament destiné au traitement de l'hyperplasie bénigne de la prostate.
